# FASCICULE DE BREVET EUROPEEN

(11) **EP 3 438 658 B1**
(45) Date de publication et mention de la délivrance du brevet: **08.12.2021**
(21) Numéro de dépôt: 18197726.5
(22) Date de dépôt: 06.10.2015
(51) Int. Cl.: G01N 29/032, B06B 3/00, G01N 29/22, C22B 9/02, C22B 21/06, G01N 33/205

(54) **EQUIPEMENT DE MESURE POUR LE CONTROLE PAR ULTRASONS D'UN METAL LIQUIDE**
MESSGERÄTE ZUR ULTRASCHALLKONTROLLE EINES FLÜSSIGMETALLS
MEASURING EQUIPMENT FOR ULTRASONIC CONTROL OF A LIQUID METAL

(30) Priorité: 07.10.2014 FR 1402257
(43) Date de publication de la demande: 06.02.2019
(62) Demande divisionnaire de: 15791690.9
(73) Titulaire: Constellium Issoire, 63500 Issoire (FR)
(72) Inventeur: ACHARD, Jean-Louis, 38220 VIZILLE (FR); LE BRUN, Pierre, 38110 SAINT JEAN DE SOUDAIN (FR)
(74) Mandataire: Constellium - Propriété Industrielle

(56) Documents cités:
- GB-A- 1 496 137
- GB-A- 1 496 247
- US-A- 3 131 514
- US-A- 3 131 515
- US-B1- 6 886 406

## Description

L'invention concerne le domaine du contrôle non destructif et de l'analyse de métal liquide par ultrasons. Plus précisément, l'invention est relative à une sonotrode en céramique, c'est-à-dire inerte vis à vis du métal liquide permettant une transmission optimisée des ultrasons aux fins de contrôle, et d'analyse.

Dans le domaine de la coulée des métaux liquides, il est de la plus haute importance de contrôler de façon précise la qualité inclusionnaire. D'elle, en effet, dépend la qualité et le taux de mise au rebut des tôles ou pièces notamment les tôles ou pièces minces, en particulier celles obtenues lors de la fabrication des récipients fermés du type boîte de boisson ou aérosol. Dans le cas des métaux liquides, cette mesure est particulièrement délicate en raison du faible nombre d'inclusions présentes dans le métal liquide, mais dont la nocivité est élevée. Généralement la qualité inclusionnaire est déterminée par le taux d'inclusions contenues dans un métal liquide et par leur taille.

Elle repose essentiellement sur des méthodes de prélèvement d'échantillons de métal liquide, dans lesquels les inclusions sont concentrées sur un filtre avant d'être observées et comptées par métallographie. Tel est le cas de la méthode dite PoDFA (acronyme anglo-saxon de Porous Disk Filtration of Aluminium) dans le cas de l'aluminium liquide, mais cette méthode est seulement indicative en taille et en nombre d'inclusions.

On connaît également dans ce même domaine une méthode, dite LiMCA (acronyme anglo-saxon de Liquid Metal Cleanliness Analysis), consistant essentiellement à échantillonner le métal liquide en continu à travers un petit orifice et à γ mesurer la variation de résistance du métal liquide à chaque passage d'inclusion. Mais cet appareil, fonctionnant selon le principe du compteur Coulter, a comme inconvénients majeurs la limitation en quantité analysée (environ 0,01% du métal coulé) et en taille détectable (entre 15 et 150 µm environ).

Plus récemment, un appareil a été développé qui utilise le débit de filtration pour donner une qualification du niveau de propreté inclusionnaire. Tel est le cas notamment de la méthode Prefil^{®}. Dans ces méthodes, les fréquences d'échantillonnages réalisables en pratique sont faibles, et la fraction volumique analysée reste dès lors faible (ordre de grandeur pratique de 0,01% du métal coulé).

Le besoin de développer une méthode de mesure en continu, notamment lorsque le métal circule dans une goulotte, remonte aux années 1960.

Reynolds a ainsi exploré la voie de la mesure ultrasonore pour évaluer la qualité inclusionnaire de l'aluminium liquide, dont on attend qu'elle permette d'accéder à une fraction beaucoup plus importante du métal liquide (plusieurs pourcent au moins). L'équipement développé à l'époque, connu sous le nom de « Reynolds 4M », avait une sensibilité limitée et n'a semble-t-il été utilisé que de manière qualitative au travers d'un indice de qualité capable de différencier le métal propre du métal très sale.

Il a fait l'objet de la demande de brevet US 4,287,755 de "Reynolds Metals Company" en 1979, et de la publication de Mansfield, T.L., "Molten Aluminum Quality Measured with Reynolds 4M System".

Dans les développements plus récents concernant les ultrasons, il faut noter l'appareil « MV20/20 » de « Metalvision », disponible sur le marché et décrit dans la publication « An ultrasonic sensor for the continuous online monitoring of the cleanliness of liquid aluminium », TMS2005. Il donne, en temps réel, une indication sur la taille et le nombre d'inclusions présentes dans le métal liquide, mais sans aucune méthode de calibration associée. Cet appareil n'est que très peu utilisé, et en tout cas pas à grande échelle, essentiellement en raison de son manque de fiabilité. On note en particulier que le guide d'ondes, ou sonotrode, est en acier, réagit avec le métal, ce qui entraîne une évolution de l'interface et donc de la qualité et du niveau de transmission des ondes.

A la fin des années 1990, la demanderesse, « Pechiney Rhenalu », a également développé la mesure des inclusions par ultrasons et déposé notamment, en 1999, la demande FR2 796 155 relative à une méthode d'étalonnage de la taille des défauts vus par ultrasons dans le métal liquide, intitulée « Procédé et dispositif améliorés de comptage des inclusions dans un bain de métal liquide par ultrasons ». Ces divers travaux, tout en fournissant une méthode de calibration, n'ont toutefois pas permis de fiabiliser la méthode ultrasonore de détection/comptage des inclusions présentes dans le métal liquide en démontrant néanmoins qu'elle permettrait d'analyser une plus grande fraction du métal liquide. Cette fiabilité restreinte est due en particulier au manque de stabilité de l'interface guide d'onde/métal liquide. Il est connu en effet que les guides d'ondes doivent être mouillés par le métal liquide pour permettre la transmission de l'énergie vers le métal liquide sans perte trop importante. Pour cette raison, les guides d'ondes utilisés sont en métal, notamment en acier ou titane. Toutefois cela ne suffit pas à l'obtention d'un mouillage parfait, et des méthodes ont été développées pour l'améliorer. En témoigne, dans le contexte de l'aluminium liquide, notamment le brevet EP0035545B1, sous priorité de 1979, de « Reynolds Metal Company » revendiquant le dépôt d'un film d'aluminium en phase vapeur sur une sonotrode en titane. Mais en fait, même dans une telle configuration, la qualité du mouillage évolue en cours d'utilisation du fait de la réaction de la matière du guide d'ondes avec le métal liquide et la sonotrode avec son dépôt n'est pas réutilisable.

La demande GB 1496 137 divulgue un procédé de soudage de pièces en plastique par application d'énergie vibratoire ultrasonore par un transducteur à ultrasons ayant une face de travail qui engage une des pièces, l'amplitude de vibration de la face de travail est surveillée par des moyens de détection fournissant un signal de sortie proportionnel à cette amplitude, le signal de sortie est comparé à un signal de référence déterminé expérimentalement et l'application d'énergie ultrasonore aux pièces est interrompue lorsque la différence entre le signal de sortie et le signal de référence atteint une valeur de référence présélectionnée. Le transducteur à ultrasons comprend une paire d'éléments piézoélectriques pris en sandwich entre des corps métalliques et auxquels est appliquée une tension alternative de fonctionnement aux bornes. Un cornet conique est fixé au corps et a une face de travail.

US 3 131 515 divulgue un appareil pour générer de l'énergie vibratoire à des vitesses de particules très élevées, comprenant une tige élastique effilée longitudinalement ayant une longueur entre les extrémités d'un nombre entier de demi-longueurs d'onde d'une onde d'énergie vibratoire de torsion d'une fréquence prédéterminée, la tige effilée ayant un diamètre maximal dépendant de la fréquence de l'énergie d'entraînement en torsion et de la vitesse des ondes de torsion dans la tige, et des moyens pour entraîner la plus grande extrémité de la tige par l'énergie d'onde vibratoire de torsion de la valeur prédéterminée de la fréquence.

US-A-6886406 décrit un appareil de détection de dépôts comprenant un appareil acoustique adapté pour opérer dans un mode de résonance. L'appareil acoustique comprend une surface de détection directement exposée aux fluides d'un puits de forage. Le dépôt de matériaux sur la surface de détection est détecté en mesurant un changement de la fréquence de résonance de l'appareil acoustique.

Les matériaux réfractaires ne sont pas utilisés dans les métaux liquides précisément parce qu'ils ne sont pas mouillés par les dits métaux liquides. Seul un procédé de dépôt chimique permettrait d'obtenir le mouillage, mais pour un temps limité, ce qui n'est ni fiable ni pratique ni économique.

Il n'existe donc pas à ce jour de méthode capable de détecter de manière fiable des inclusions dans une fraction importante du métal coulé.

Un des buts de la présente invention consiste alors à pallier au moins l'un des inconvénients précités. A cet effet, la présente invention propose un équipement de mesure selon la revendication 1 utilisé pour le contrôle par ultrasons d'un aluminium liquide.

Le procédé de contrôle par ultrasons d'un métal liquide comprend les étapes suivantes :
a) Fournir une sonotrode (1) formée en céramique, et de préférence un nitrure de silicium ou un oxynitrure de silicium, tel que le SIALON, ou un métal substantiellement inerte audit métal liquide,
b) Plonger au moins en partie la sonotrode (1) dans un bain dudit métal,
c) Appliquer à la sonotrode (1) des ultrasons de puissance d'une puissance supérieure à 10 watts pour obtenir le mouillage de ladite sonotrode par ledit métal,
d) Appliquer de façon continue à la sonotrode (1) des ultrasons de mesure, appelés aussi ultrasons de contrôle, dont la fréquence est comprise entre 1 et 25 MHz,
e) Appliquer de façon intermittente à la sonotrode (1) des ultrasons de puissance d'une puissance supérieure à 10 watts, pour maintenir ledit mouillage et garantir la stabilité du signal dans le temps.

Ainsi, le mouillage de la sonotrode par le métal liquide est maintenu de façon durable, notamment sur plusieurs jours, par simples applications d'ultrasons de puissance à la sonotrode plongée dans le métal liquide. A l'étape b) la sonotrode est plongée dans un bain de métal liquide.

Des ultrasons de puissance sont appliqués à la sonotrode à l'étape c) et permettent l'obtention du mouillage par le métal liquide. Grâce à ce procédé, il est alors possible d'obtenir une transmission optimisée des ultrasons au métal liquide durable et stable dans le temps.

Avantageusement, l'application des ultrasons de puissance à l'étape e) est réalisée de façon intermittente. Le mouillage de la sonotrode résiste en effet au cours du temps de sorte que l'application d'ultrasons de puissance pour le maintien du mouillage peut être sporadique.

Quant à l'étape d), elle comprend en outre l'application d'ultrasons de mesure à la sonotrode dont la fréquence est comprise entre 1 et 25 MHz. Il est alors possible d'utiliser la sonotrode préalablement mouillée à l'étape c) pour des applications telles que le contrôle non destructif du métal liquide par application d'ultrasons de mesure à la sonotrode sur une longue période.

Ainsi, la mesure par ultrasons est effectuée en continu de sorte à pouvoir analyser en ligne la qualité inclusionnaire du métal liquide et notamment dans une goulotte, avant son traitement ou avant une coulée dans une lingotière. L'invention permet en effet l'application concomitante d'ultrasons de mesure pour le traitement et d'ultrasons de puissance intermittents pour la régénération du mouillage, celui-ci ayant tendance à se détériorer dans le temps, par simple effet de maintien prolongé ou par la présence au contact de la sonotrode de gaz, oxydes ou autres impuretés.

Selon un mode de réalisation avantageux, le métal liquide est un alliage d'aluminium liquide que nous appellerons par la suite aluminium liquide.

Cet alliage d'aluminium peut contenir du magnésium à une teneur Y non nulle et même très faible, de l'ordre de 20 ppm. Selon un autre mode de réalisation, la teneur Y est supérieure ou égale à 0,05 %, de préférence supérieure à 0,5 %, et mieux encore supérieure ou égale à 0,7 % en poids.

Toutefois le métal liquide peut aussi être du sodium, du zinc, ou autre métal, et la sonotrode en céramique et en particulier un nitrure de silicium ou un oxynitrure de silicium, tel que le SIALON.

Avantageusement, le procédé comprend avant l'étape d), une étape comprenant la disposition, dans le métal liquide, d'un réflecteur d'étalonnage, de préférence inerte vis-à-vis dudit métal, et l'étape d) comprend une étape de réflexion par le réflecteur des ultrasons de mesure transmis par la sonotrode, de sorte à générer un signal ultrasonore, le procédé comprenant une étape de déclenchement d'application des ultrasons de puissance selon l'étape d) lorque le signal ultrasonore généré présente une intensité inférieure ou égale à une intensité seuil prédéterminée.

Selon un mode préférentiel du procédé, le métal liquide est de l'aluminium liquide. Selon un autre mode de réalisation du procédé, le métal liquide est du sodium, du zinc, ou autre métal, et la sonotrode en céramique et en particulier un nitrure de silicium ou un oxynitrure de silicium, tel que le SIALON.

Ainsi lorsqu'au cours du temps, le mouillage de la sonotrode diminue et les ultrasons sont véhiculés dans le bain avec une intensité réduite, l'intensité du signal ultrasonore réfléchi par le réflecteur diminue, automatiquement l'application d'ultrasons de puissance est déclenchée de sorte à régénérer le mouillage de la sonotrode par le métal liquide sur une longue période et ainsi stabiliser la transmission des ultrasons dans le temps.

Selon une possibilité, l'application des ultrasons de puissance de l'étape e) est déclenchée de façon périodique, notamment avec une périodicité comprise entre quelques secondes et quelques heures, typiquement entre 30 minutes et 6 heures. Cette configuration permet en effet de régénérer le mouillage de la sonotrode pour une utilisation sur une longue période avec un signal stable dans le temps.

De préférence l'application des ultrasons de puissance selon l'étape e) est réalisée pendant une durée de quelques secondes à quelques minutes, typiquement environ une minute. Cette durée suffit en effet à maintenir le mouillage dans d'excellentes conditions.

Le procédé comprend, avant l'étape e), une étape de fixation d'un transducteur d'émission d'ultrasons de mesure à la sonotrode selon un montage axial. Ce type de montage permet avantageusement d'obtenir une émission ou une réception directionnelle des ondes acoustiques.

De même, avantageusement, le procédé comprend, avant les étapes c) et e) une étape de montage axial d'un transducteur d'émission d'ultrasons de puissance sur ladite sonotrode en addition du transducteur d'émission d'ultrasons de mesure ou contrôle. Ainsi les transducteurs de mesure et de puissance sont fixés sur la même sonotrode selon un montage axial.

Selon une possibilité, la fixation est obtenue par collage du transducteur de mesure à la sonotrode éventuellement via une bride. Selon une autre possibilité la fixation est obtenue par vissage du transducteur sur la bride cylindrique liée intimement à la sonotrode. Cette configuration permet d'assurer un couplage mécanique durable entre les transducteurs et la sonotrode pour des mesures de qualité de l'aluminium liquide, telles que la détection d'inclusions, la vélocimétrie Doppler aux ultrasons, l'hydrophonie dans le métal liquide.

Selon un autre possibilité la bride est assemblée à la sonotrode par « brasage » et dans ce dernier cas, la sonotrode est préalablement immergée partiellement dans un bain d'aluminium liquide comprenant au moins 0.05 % en poids de magnésium, est soumise à des ultrasons de puissance jusqu'à l'obtention du mouillage de la sonotrode par l'aluminium liquide, puis l'aluminium liquide est refroidi et solidifié avec formation d'une liaison intime entre la sonotrode et l'aluminium qui est ensuite usiné sous forme d'une bride.

La bride ainsi formée présente une énergie de liaison avec la sonotrode ayant des propriétés similaires à celles obtenues lors d'un brasage entre deux métaux. Une section polie de l'interface obtenue par ce procédé entre la sonotrode liée au métal, observée par microscope électronique à balayage (MEB) montre en effet un scellement avec une liaison parfaite, sans aucune décohésion et une continuité entre les deux matériaux du type à permettre un couplage mécanique optimal entre le métal et la sonotrode. Ainsi, la liaison intime entre la sonotrode et la bride présente une force de liaison au moins sensiblement égale à celle d'un brasage entre les deux matériaux, c'est-à-dire qu'il est impossible de détacher la bride de la sonotrode sans arrachement de matière.

Selon une possibilité de réalisation, le métal liquide est en mouvement. Avantageusement, l'étape d) comprend la mesure par vélocimétrie doppler aux ultrasons du métal liquide.

Selon une autre possibilité, l'étape d) comprend la détection et la mesure d'inclusions dans le métal liquide . Cette détection peut avoir lieu en ligne ou à poste fixe.

Selon un mode de réalisation, le métal liquide est de l'aluminium liquide.

Selon un autre mode, ledit métal liquide est du sodium ou du zinc, et la sonotrode en acier ou autre métal non mouillé par respectivement le sodium ou le zinc, ou en Sialon.

D'autres aspects, buts et avantages de la présente invention apparaîtront mieux à la lecture de la description suivante de trois modes de réalisation de celle-ci, donnée à titre d'exemple non limitatif et faite en référence aux dessins annexés. Les figures ne respectent pas nécessairement l'échelle de tous les éléments représentés de sorte à améliorer leur lisibilité. Dans la suite de la description, par souci de simplification, des éléments identiques, similaires ou équivalents des différentes formes de réalisation portent les mêmes références numériques.
Les figures 1 à 3 illustrent schématiquement la fabrication d'un équipement comprenant une sonotrode pour la réalisation d'un mode de réalisation du procédé selon l'invention.
La figure 4 illustre un premier mode de réalisation de l'invention.
Les figures 5 et 6 illustrent un deuxième mode de réalisation de l'invention.
La figure 7 illustre un troisième mode de réalisation de l'invention.

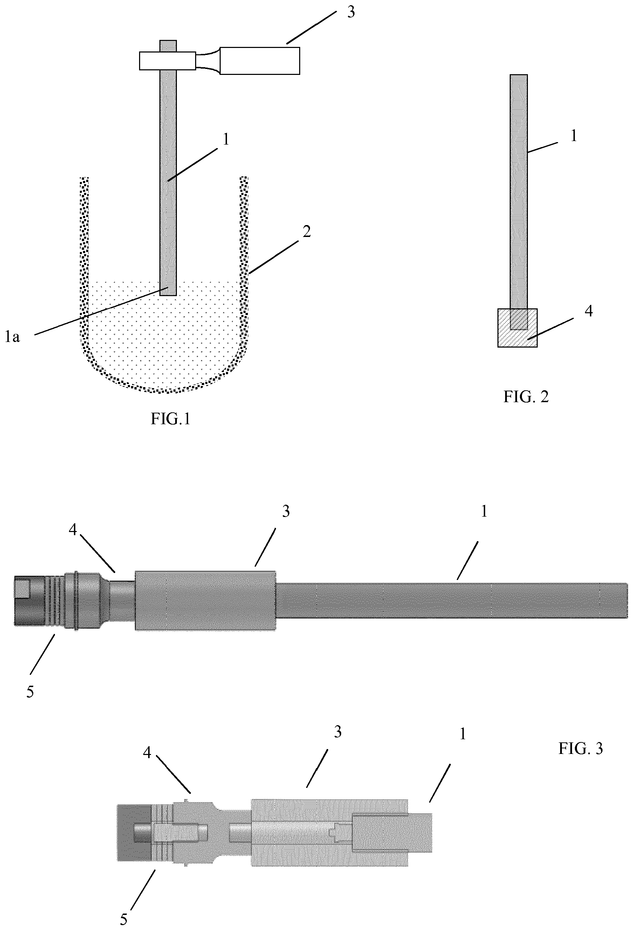

Comme illustré aux figures 1 à 3, une sonotrode 1 de Si₃N₄ est mouillée dans un bain d'alliage d'aluminium liquide en vue d'y être utilisée avec un mouillage maintenu.

La figure 1 représente l'insertion d'une première région d'extrémité la de la sonotrode 1 cylindrique, cette dernière présentant une longueur de 400 mm et un diamètre de 30 mm, dans un creuset 2 contenant 3 kg d'aluminium liquide, en l'occurrence de type Al5%Mg comportant environ 5 % de magnésium en poids.

Un transducteur d'émission d'ultrasons de puissance 3 assemblé à la sonotrode 1 selon un mode latéral transmet des ultrasons de puissance d'une fréquence de 19,8 kHz et d'une puissance d'environ 150 W à la sonotrode 1 pendant 5 minutes.

Lorsque l'on retire la sonotrode 1 du bain à cette étape du procédé, celle-ci montre un mouillage parfait par l'aluminium liquide, identifiable à l'œil nu grâce sa couleur gris clair brillant, caractéristique de l'aluminium, et surtout non séparable de la surface céramique à l'aide d'un outil sans arrachement de matière.

Bien entendu, d'autres conditions de mouillage peuvent être utilisées, avec une teneur notamment en magnésium plus ou moins importante, et de préférence avec une teneur minimale de 0.05 % en poids.

Les ultrasons de puissance sont adaptés en conséquence de la teneur en magnésium utilisée. Ils sont notamment appliqués plus ou moins longtemps de sorte à obtenir la cavitation dans l'aluminium liquide générant le mouillage de la sonotrode 1.

Selon une possibilité non illustrée, l'application des ultrasons est effectuée l'aide d'un transducteur d'ultrasons de puissance 3 fixé à la sonotrode 1 selon un mode axial, par serrage ou par collage ou par vissage ou toute autre possibilité dans la mesure où la fixation résiste sur la durée de l'opération, typiquement inférieure à 15 min.

A l'étape suivante l'aluminium liquide est refroidi autour de la sonotrode 1 mouillée (la sonotrode 1 n'a pas été retirée du bain pour vérifier le mouillage dans ce cas). L'aluminium se solidifie et conduit à la formation d'une liaison intime autour la sonotrode 1. L'aluminium solidifié est alors usiné sous la forme d'une bride 4 cylindrique autour de la sonotrode 1.

Comme représenté à la figure 3, un transducteur d'émission d'ultrasons de puissance 3 et un transducteur d'émission d'ultrasons de mesure 5 sont vissés sur la bride 4 en aluminium selon un montage axial.

La deuxième région d'extrémité 1b de la sonotrode 1, opposée à la première région d'extrémité la fixée à la bride 4 est alors plongée dans un alliage d'aluminium, et des ultrasons de puissance sont appliqués afin d'obtenir le mouillage de cette deuxième région d'extrémité 1b (étape c).

Une fois la sonotrode mouillée par l'aluminium liquide, des ultrasons de mesure sont appliqués par l'intermédiaire du transducteur d'émission d'ultrasons de mesure 5. Ces ultrasons appliqués avec une fréquence de 5 MHz permettent notamment d'analyser la qualité inclusionnaire (quantification et tailles des inclusions) dans l'alliage d'aluminium liquide de façon continue, notamment sur plusieurs heures (étape d).

Des ultrasons sont appliqués avec une puissance supérieure à 10 W, par l'intermédiaire de la bride 4 en aluminium (étape e). Ceci assure la régénération du mouillage dans l'alliage d'aluminium liquide. Une hypothèse que l'on peut formuler réside en ce que, au cours de l'utilisation de la sonotrode en mode contrôle ou mesure, une pellicule d'oxyde se forme à la surface et altère le mouillage. Il est supposé que la nouvelle application d'ultrasons permet de fragmenter cette pellicule d'oxyde et de régénérer le contact aluminium liquide / aluminium de mouillage de la sonotrode une fois la sonotrode 1 plongée dans l'alliage. L'hypothèe de l'accumulation de gaz au voisinage de la sonotrode n'est pas non plus à exclure.

Des ultrasons de puissance sont ainsi émis avec une fréquence d'environ 20kHz de façon périodique, notamment toutes les 3 heures pendant une durée d'environ 1 minute de sorte à maintenir le mouillage de la sonotrode 1.

Il semble en effet que l'application périodique d'ultrasons de puissance permet de « nettoyer » l'interface sonotrode 1 - aluminium liquide de toute inclusion ou bulle de gaz qui s'y serait déposée au cours de l'utilisation de la sonotrode dans l'aluminium liquide.

Le processus est analogue dans le cas ou le métal liquide est du sodium ou du zinc, et la sonotrode en céramique, notamment du Sialon.

Selon une autre possibilité non illustrée, les ultrasons sont émis de façon intermittente, en fonction de l'intensité du signal reçu en retour lorsqu'un réflecteur d'étalonnage des ultrasons de mesure est utilisé dans le bain contenant l'alliage.

Il est ainsi possible d'utiliser ainsi la sonotrode 1 pour différentes applications.

Des mesures de la qualité inclusionnaire peuvent notamment être réalisées en continu, sur la durée d'au moins une coulée, et sur une grande fraction de volume, du fait, en particulier, de l'excellente transmission des ultrasons dans le métal liquide .

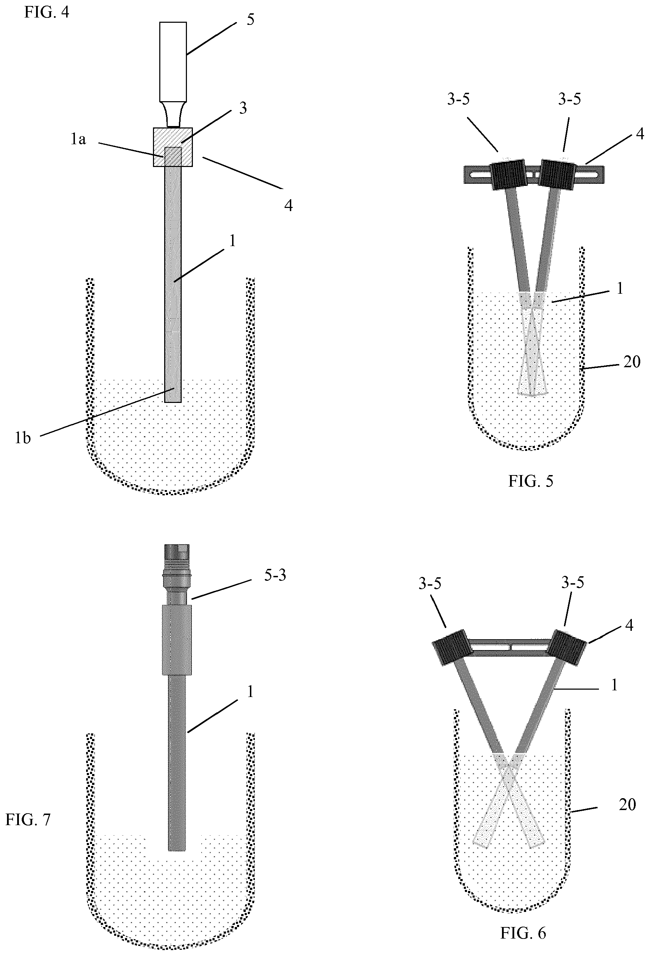

Les figures 5 et 6 illustrent un mode de réalisation du procédé appliqué en particulier à la mesure de la qualité inclusionnaire d'un alliage d'aluminium liquide, mais applicable à tout métal liquide et sonotrode inerte vis-à-vis de cette dernière. Deux sonotrodes 1 en Si3N4 (longueur 400 mm diamètre 30 mm), mouillées par application préalable d'ultrasons de puissance, sont ici partiellement immergées dans un creuset 20 comprenant 25 kg d'alliage d'aluminium liquide. Le transducteur d'ultrasons de puissance 3 est fixé selon un montage axial sur la bride 4 cylindrique sur chacune d'entre elles. Un transducteur d'émission d'ultrasons de mesure 5 est disposé dans la bride 4 au contact du barreau d'une sonotrode 1 de Si3N4.

La sonotrode 1, sur laquelle le transducteur d'émission d'ultrasons de mesure 5 est fixé, est utilisée pour l'émission des ultrasons de mesure tandis que l'autre sonotrode 1 est utilisée en mode réception. Le montage en tandem des deux sonotrodes 1 permet, en modifiant l'angle et l'écart entre les sonotrodes 1, d'obtenir une focalisation géométrique du faisceau ultrasonore. Un écart petit entre les sonotrodes 1 et un angle α faible permettent d'augmenter le volume d'alliage d'aluminium contrôlé mais la limite de détection est augmentée en termes de taille d'inclusion de sorte que la détection présente en fait une sensibilité moins importante (figure 5). A l'inverse, un grand écart entre les sonotrodes 1 et un angle α important permettent de diminuer le volume contrôlé et la limite de détection est abaissée (figure 6). Dans ce dernier cas, la sensibilité de détection est plus importante.

Après introduction des sonotrodes 1 dans l'alliage d'aluminium liquide, avec un écart de 300 mm et un angle α de 28°, le signal ultrasonore de mesure n'est pas significativement présent. L'alliage d'aluminium liquide A ne mouille pas les sonotrodes 1. L'application d'ultrasons de puissance (19.8 kHz, 150 W, 5 secondes) sur la sonotrode 1 fonctionnant en mode émission puis l'application d'ultrasons de puissance sur la sonotrode 1 fonctionnant en mode réception permet d'établir le mouillage : il γ a alors ensuite transmission du signal ultrasonore de mesure (5MHz). Dans ce cas, le niveau de bruit détecté augmente et des pics qui correspondent à des particules unitaires (inclusions) apparaissent. L'application régulière d'ultrasons de puissance permet de maintenir le mouillage et une continuité dans la détection et la quantification des inclusions, encore connue sous le nom de mesure de la propreté inclusionnaire, dans un bain d'alliage d'aluminium liquide en ligne ou à poste fixe.

Selon un autre mode d'exécution du procédé illustré à la figure 7, la sonotrode 1 est utilisée aux fins de vélocimétrie ultrasonore par effet Doppler d'un bain d'alliage d'aluminium liquide en mouvement, ce qui était jusqu'à présent limité de manière fiable aux métaux à bas point de fusion.

Dans ce mode de réalisation, un transducteur d'ultrasons de puissance 3 et un transducteur d'émission d'ultrasons de mesure 5 sont fixés selon un montage axial sur une sonotrode 1 en SIALON et des ultrasons de puissance (20 kHz, 120 W, 8 s) sont appliqués pour établir le mouillage.

Des ultrasons de mesure, de mêmes caractéristiques que précédemment, sont appliqués en continu et l'évolution de la fréquence du signal ultrasonore est mesurée grâce au transducteur 3 qui fonctionne également en réception. En parallèle, les ultrasons de puissance sont appliqués toutes les cinq heures pour maintenir le mouillage de la sonotrode 1 (étape e). L'évolution de la fréquence réfléchie par les particules en suspension, par rapport à la fréquence émise, est à l'image du déplacement des particules entraînées par le fluide.

Ainsi, la présente invention propose un équipement de mesure comprenant une sonotrode 1 mouillée par de l'aluminium liquide, applicable à une méthode de mesure qui comprend l'utilisation en continu d'un appareil de mesure des inclusions dans un alliage d'aluminium liquide, qui offre la possibilité de passer d'un mode mesure (ultrasons de mesure) à un mode régénération du mouillage (ultrasons de puissance) sans devoir intervenir sur l'appareil, qui reste immergé et est autonome.

De plus, les sonotrodes 1 ne nécessitent aucun traitement de surface pour modifier chimiquement la surface du matériau réfractaire constituant la sonotrode 1.

Il est alors possible d'utiliser ce procédé pour un contrôle non destructif en continu de l'alliage d'aluminium liquide et pour l'analyse, notamment de la qualité inclusionnaire de l'aluminium grâce à la régénération du mouillage.

L'équipement de mesure utilisé pour le procédé comprend un montage dans lequel la sonotrode 1 et les transducteurs ultrasonores 3, 5 (mesure et puissance) constituent une seule entité.

De la même façon, le procédé peut être utilisé dans le cas où dans lequel ledit métal liquide est du sodium ou du zinc, et la sonotrode en céramique, notamment en Sialon.

## Revendications

1. Equipement de mesure pour le contrôle par ultrasons d'un aluminium liquide, comprenant une sonotrode (1) en céramique, un transducteur d'émission d'ultrasons de puissance (3) dont la puissance est supérieure à 10 Watts et un transducteur d'émission d'ultrasons de mesure (5) dont la fréquence est comprise entre 1 et 25 MHz **caractérisé en ce que** le dit équipement de mesure comprend un montage dans lequel la sonotrode (1), le transducteur d'émission d'ultrasons de puissance (3) et le transducteur d'émission d'ultrasons de mesure (5) constituent une seule entité de telle sorte à ce que la sonotrode (1) transmette les ondes émises par le transducteur d'émission d'ultrasons de puissance (3) et le transducteur d'émission d'ultrasons de mesure (5).

2. Equipement de mesure pour le contrôle par ultrasons d'un aluminium liquide selon la revendication 1, **caractérisée en ce que** les transducteurs de mesure (5) et de puissance (3) sont fixés sur la sonotrode (1) selon un montage axial.

3. Equipement de mesure pour le contrôle par ultrasons d'un aluminium liquide selon la revendication 2, **caractérisée en ce que** le transducteur de mesure est fixé à la sonotrode (1) par collage.

4. Equipement de mesure pour le contrôle par ultrasons d'un aluminium liquide selon la revendication 3, **caractérisée en ce que** le transducteur de mesure (5) est fixé à la sonotrode (1) par collage via une bride.

5. Equipement de mesure pour le contrôle par ultrasons d'un aluminium liquide selon la revendication 2, **caractérisée en ce que** le transducteur de mesure (5) est fixé à la sonotrode (1) par vissage sur une bride cylindrique liée intimement liée à la sonotrode (1).

6. Equipement de mesure pour le contrôle par ultrasons d'un aluminium liquide selon la revendication 4 ou 5 **caractérisée en ce que** la bride est fixée à la sonotrode (1) par brasage et dans ce cas, la sonotrode est préalablement immergée partiellement dans un bain d'aluminium liquide comprenant au moins 0.05 % en poids de magnésium, est soumise à des ultrasons de puissance jusqu'à l'obtention du mouillage de la sonotrode par l'aluminium liquide, puis l'aluminium liquide est refroidi et solidifié avec formation d'une liaison intime entre la sonotrode et l'aluminium qui est ensuite usiné sous forme d'une bride.

7. Equipement de mesure pour le contrôle par ultrasons d'un aluminium liquide selon l'une quelconque des revendications 1 à 6 , **caractérisée en ce que** la sonotrode (1) est en nitrure de silicium ou en oxynitrure de silicium, tel que le Sialon.

## Patentansprüche

1. Messausrüstung zur Ultraschallkontrolle eines flüssigen Aluminiums, die eine Sonotrode (1) aus Keramik, einen Transducer zum Senden von Leistungs-Ultraschallwellen (3), deren Leistung über 10 Watt beträgt, und einen Transducer zum Senden von Mess-Ultraschallwellen (5), deren Frequenz zwischen 1 und 25 MHz liegt, umfasst, **dadurch gekennzeichnet, dass** die Messausrüstung eine Montage umfasst, in welcher die Sonotrode (1), der Transducer zum Senden von Leistungs-Ultraschallwellen (3) und der Transducer zum Senden von Mess-Ultraschallwellen (5) eine einzige Einheit darstellen, so dass die Sonotrode (1) die von dem Transducer zum Senden von Leistungs-Ultraschallwellen (3) und dem Transducer zum Senden von Mess-Ultraschallwellen (5) gesendeten Wellen überträgt.

2. Messausrüstung zur Ultraschallkontrolle eines flüssigen Aluminiums nach Anspruch 1, **dadurch gekennzeichnet, dass** der Messtransducer (5) und der Leistungstransducer (3) gemäß einer axialen Montage auf der Sonotrode (1) befestigt sind.

3. Messausrüstung zur Ultraschallkontrolle eines flüssigen Aluminiums nach Anspruch 2, **dadurch gekennzeichnet, dass** der Messtransducer an der Sonotrode (1) durch Kleben befestigt ist.

4. Messausrüstung zur Ultraschallkontrolle eines flüssigen Aluminiums nach Anspruch 3, **dadurch gekennzeichnet, dass** der Messtransducer (5) an der Sonotrode (1) durch Kleben mittels eines Flanschs befestigt ist.

5. Messausrüstung zur Ultraschallkontrolle eines flüssigen Aluminiums nach Anspruch 2, **dadurch gekennzeichnet, dass** der Messtransducer (5) an der Sonotrode (1) durch Schrauben auf einem zylindrischen Flansch befestigt ist, der eng mit der Sonotrode (1) verbunden ist.

6. Messausrüstung zur Ultraschallkontrolle eines flüssigen Aluminiums nach Anspruch 4 oder 5, **dadurch gekennzeichnet, dass** der Flansch an der Sonotrode (1) durch Löten befestigt ist und in diesem Fall die Sonotrode zuvor in ein flüssiges Aluminiumbad getaucht wird, das mindestens 0,05 Gew.-% Magnesium umfasst, Leistungs-Ultraschallwellen bis zum Erhalt der Benetzung der Sonotrode durch das flüssige Aluminium ausgesetzt wird, dann das flüssige Aluminium mit Ausbildung einer engen Verbindung zwischen der Sonotrode und dem Aluminium abgekühlt und verfestigt wird, das dann in Form eines Flanschs bearbeitet wird.

7. Messausrüstung zur Ultraschallkontrolle eines flüssigen Aluminiums nach einem der Ansprüche 1 bis 6, **dadurch gekennzeichnet, dass** die Sonotrode (1) aus Siliciumnitrid oder aus Siliciumoxinitrid wie Sialon ist.

## Claims

1. A measuring equipment for ultrasonic control of liquid aluminium, comprising a ceramic sonotrode (1), a power ultrasound emission transducer (3) whose power is greater than 10 Watts and a measurement ultrasound emission transducer (5) whose frequency is comprised between 1 and 25 MHz **characterised in that** said measuring equipment comprises an assembly wherein the sonotrode (1), the power ultrasound emission transducer (3) and the measurement ultrasound emission transducer (5) constitute a single entity so that the sonotrode (1) transmits the waves emitted by the power ultrasound emission transducer (3) and the measurement ultrasound emission transducer (5).

2. The measuring equipment for ultrasonic control of liquid aluminium according to claim 1, **characterised in that** the measurement (5) and power (3) transducers are fixed on the sonotrode (1) in an axial mounting.

3. The measuring equipment for ultrasonic control of liquid aluminium according to claim 2, **characterised in that** the measurement transducer is fixed to the sonotrode (1) by bonding.

4. The measuring equipment for ultrasonic control of liquid aluminium according to claim 3, **characterised in that** the measurement transducer (5) is fixed to the sonotrode (1) by bonding via a flange.

5. The measuring equipment for ultrasonic control of liquid aluminium according to claim 2, **characterised in that** the measurement transducer (5) is fixed to the sonotrode (1) by screwing onto a cylindrical flange closely bonded to the sonotrode (1).

6. The measuring equipment for ultrasonic control of liquid aluminium according to claim 4 or 5 **characterised in that** the flange is fixed to the sonotrode (1) by soldering and in this case, the sonotrode is partially immersed beforehand in a liquid aluminium bath comprising at least 0.05% by weight of magnesium, is subjected to power ultrasound until the sonotrode is wetted by liquid aluminium, then the liquid aluminium is cooled and solidified with the formation of a close bond between the sonotrode and the aluminium which is then machined in the shape of a flange.

7. The measuring equipment for ultrasonic control of liquid aluminium according to any one of claims 1 to 6, **characterised in that** the sonotrode (1) is made of silicon nitride or of silicon oxynitride, such as Sialon.
